Europäisches Patentamt

European Patent Office

Office européen des brevets

(11) Publication number: **0 168 096**
**B1**

# EUROPEAN PATENT SPECIFICATION

(45) Date of publication of patent specification: **18.05.88**

(51) Int. Cl.⁴: **B 01 J 23/74,** B 01 J 23/72, C 11 C 3/12, C 07 C 85/12

(21) Application number: **85201010.7**

(22) Date of filing: **26.06.85**

(54) **Hydrogenation catalysts.**

(30) Priority: **02.07.84 NL 8402096**

(43) Date of publication of application:
**15.01.86 Bulletin 86/03**

(45) Publication of the grant of the patent:
**18.05.88 Bulletin 88/20**

(84) Designated Contracting States:
**AT BE CH DE FR GB IT LI NL SE**

(56) References cited:
**EP-A-0 013 275**
**EP-A-0 076 602**
**DE-A-1 958 952**
**FR-A-2 256 780**
**FR-A-2 373 330**
**US-A-4 146 504**
**US-A-4 174 300**

The file contains technical information
submitted after the application was filed and
not included in this specification

(73) Proprietor: **UNILEVER NV**
**Burgemeester s'Jacobplein 1 P.O. Box 760**
**NL-3000 DK Rotterdam (NL)**
(84) **BE CH DE FR IT LI NL SE AT**

(73) Proprietor: **UNILEVER PLC**
**Unilever House Blackfriars P.O. Box 68**
**London EC4P 4BQ (GB)**
(84) **GB**

(72) Inventor: **Oudejans, Johannes Cornelis**
**Hendrik Tollensstraat 56**
**NL-2624 BH Delft (NL)**
Inventor: **Dekker, Jan Gerrit**
**Bomendijk 11**
**NL-3181 RE Rozenburg (NL)**
Inventor: **Klauenberg, Günter**
**Hügelweg 5**
**D-4190 Kleve-Materborn (DE)**
Inventor: **Klimmek, Helmut**
**Florastrasse 50**
**D-4150 Krefeld (DE)**

(74) Representative: **Wiesenhaan, Herman, Drs. et al**
**Unilever N.V. Patent Division P.O.Box 137**
**NL-3130 AC Vlaardingen (NL)**

Courier Press, Leamington Spa, England.

## Description

The application relates to a hydrogenation catalyst which contains 10—90 parts by weight of metal/metal compounds and 90—10 parts of silica and has a total active metal surface area of 1 to 25, perferably more than 2 m²/g of metal, in which the metal has an uneven atomic number between 26 and 30 (which is to say that the active metal is cobalt or copper).

Similar catalysts are already known in the art and are used for various hydrogenation reactions. Cobalt catalysts are especially effective for the hydrogenation of nitriles to amines and copper catalysts are especially effective for the selective hydrogenation of semi-drying oils.

Cobalt-silica catalysts are disclosed inter alia in EP—A—0 013 275. There the preparation takes place by co-precipitation of cobalt ions and silicate ions on a porous carrier with an alkaline precipitation agent. In this method of preparation the precipitation time is typically about 20 minutes.

However, these catalysts can only be filtered with difficulty and their catalytic properties (combination of selectivity and activity) need improvement.

Copper-silica catalysts are disclosed inter alia in US—A—4,174,300. The preparation takes place by coprecipitation, preferably by slowly adding an aqueous solution of a copper salt (e.g. nitrate) dropwise to a silicate solution. Carrier material can be present.

Copper-silica catalysts are also disclosed in GB—A—2,121,310, they are prepared by adding silica to a copper-ammonia complex and subsequently removal of the amounts by boiling for in a few hours at temperatures up to 100°C. In this method the copper ions are precipitated on the silica.

Finally there is also European patent application (EP—A—)84200028/114704 (Unilever) which is not a prior publication (01.08.84) which describes nickel-silica catalysts, in which, the nickel compounds are for the greater part precipitated next to the silica, so that the nickel/nickel compound particles are for at least 60% free of carrier particles. (Nickel has atomic number 28.)

The present invention provides metal catalysts in which the active metal has an uneven atomic number between 26 and 30, (that is to say cobalt-and/or copper)-silica catalysts having improved properties as to speed of filtration and hydrogenation, which catalyst contains metal/metal compound aggregates of which the average particle size is between 5 and 50, preferably below 40 micrometers and the metal/metal compound aggregates are for at least 60% free of carrier particles. Preferably, the surface area of the metal/metal compound aggregates is for at least 80% free of carrier particles, more preferably for at least 90% free of carrier particles.

The average particle size of the metal/metal compound aggregates is between 10 and 30 micrometers.

The metal crystallite size is, on an average, between 10 and 25 nanometers, preferably between 14 and 20 nanometers.

Catalysts according to the invention containing a metal having an uneven atomic number between 26 and 30, a water-insoluble carrier and optionally a promotor can be prepared according to a process which is carried out in at least two separate steps, namely:

I. A rapid precipitation step in which, under vigorous mixing, metal hydroxide/carbonate is precipitated in a precipitation reactor in which the average residence time is 0.1 to 10 minutes, preferably between 0.2 and 4.5 minutes, more preferably less than 1.5 minutes, the normality of the solution in the reactor, which contains excess alkali, being between 0.05 and 0.5 M, preferably between 0.1 and 0.3 M, and the temperature of the liquid in the precipitation reactor being kept between 5 and 95, preferably between 15 and 55°C.

II. At least one separate, longer, ageing step with an average residence time in the ageing reactor of 20 to 180 minutes, preferably between 30 and 120 minutes, and a temperature between 60 and 100, preferably between 90 and 97°C, whereafter, by processes known in the art, the solid material is collected, dried and activated with hydrogen.

Preferably the solution of the metal salt contains 10—80 g metal per litre in the form of a soluble salt. The carrier is added in an amount of 20—200 g per litre.

Preferably the solution of the alkaline compound contains 30 to 300 g alkaline compound per litre (calculated as anhydrous material).

The cobalt- and/or copper-containing catalysts according to this invention contain a water-insoluble carrier which is present or is added during the preparation of the catalyst. Suitable carrier materials are silica-containing materials e.g. kieselguhr, and silicates such as bentonite. Kieselguhr or diatomaceous earth is preferred, particularly kieselguhr containing 50—90% by weight of amorphous silica.

The carrier material can be added (a) directly as such, (b) as an aqueous suspension, (c) preferably as a suspension in an aqueous metallic salt solution, (d) as a suspension in an aqueous solution of the alkaline material.

According to embodiments (a) to (d), the carrier can be added before or during the precipitation. According to embodiment (a), (b) or (d), however, the carrier can also be added completely or partly (preferably the latter) after the precipitation, but also before or during the maturing.

After precipitation and ageing according to the invention, the solid components are separated from the liquid, if desired washed, dried and activated by contacting with hydrogen at elevated temperatures.

Metal compounds which can be used as starting material for the preparation of the catalysts according to this invention are water-soluble metal compounds such as nitrates, sulphates, acetates, chlorides and formates. A solution thereof, preferably containing between 10 and 80 g of metal salt per litre, is fed to a precipitation reactor. Preferably the solution contains between 25 and 60 g of metal salt per litre.

Alkaline compounds which can be used as starting material in the method of preparation according to the present invention are alkali metal hydroxides, alkali metal carbonates, alkali metal bicarbonates, the corresponding ammonium compounds and mixtures of the above-mentioned compounds. The concentration of the alkaline solution which is fed into the precipitation reactor is preferably 20 to 300 g anhydrous material per litre (in so far as the solubility allows that), more particularly between 50 and 250 g per litre.

It is convenient to use both solutions (of metal salt and alkaline compound) in almost the same concentrations (expressed in equivalents), so that approximately the same volumes can be reacted.

The metal-containing solution and the alkaline solution are added in such amounts per unit of time that a small excess of alkaline compound is present during the precipitation step, so that the normality of the liquid is between 0.05 and 0.5, preferably between 0.1 and 0.3 (this normality is determined by titrating a solution of hydrochloric acid, with methyl-orange as indicator). Sometimes it is necessary to add some more alkaline solution during the ageing step, in order to keep the normality within the range indicated above.

The precipitation reactor has such dimensions with respect to the amounts of liquid pumped in, that the indicated short average residence times can be obtained. As a rule, average residence times of between 0.1 sec, and 10 minutes, preferably between 0.2 sec, and 4.5 minutes are used in the precipitation reactor.

In a preferred embodiment, in which the precipitation step (step 1) is carried out continuously, the amounts of solutions fed into the precipitation reactor are controlled by measuring, optionally continuously, the normality or pH of the reactor effluent. The temperature at which the precipitation takes place can be controlled by adjusting the temperatures of the liquids fed in. The required vigorous agitation of the liquid in the precipitation reactor preferably takes place with a mechanical energy input of between 5 and 2000 watts per kg of solution. More preferably the agitation takes place with a mechanical energy input of 100 to 2000 watts per kg of solution.

The reaction mixture obtained from the precipitation reactor goes immediately thereafter to a stirred post-reactor of a significantly higher capacity. Optionally, further components are added here, such as carrier material, alkaline solution as described above and/or possible promotors.

Preferably the liquid in the ageing reactor, thus during the ageing step, is kept at a temperature between 60 and 100°C, preferably between 90 and 98°C.

The precipitation step and also the maturing step can be carried out batchwise (=discontinuously), continuously and semi-continuously (e.g. according to the cascade method).

Usually the normality of the liquid in the ageing reactor during the ageing step (step 2) is kept in the same range as during the precipitation step (step 1); if necessary by adding some more alkali. The ageing step can be carried out in one or more reactors, the (total average residence time being maintained between 20 and 180 minutes, preferably between 60 and 150 minutes. If two or more reactors are used, it is preferred to have the temperature in the second or further reactor 10 to 15 centigrades lower than in the first ageing reactor.

After the ageing step has been completed, the solid material is separated from the mother liquor, usually washed, dried, optionally ground and calcined and thereafter activated with hydrogen at an elevated temperature, between 150 and 500, preferably between 200 and 400°C. This activation can take place at atmospheric pressure or at increased pressure.

Preferably before drying, or during a step preceding that, promotors can be added. Suitable amounts of promotors are from 0.5 to 10%, calculated on the weight of cobalt and/or copper, of elements such as molybdenum, nickel, iron, silver, magnesium, or other elements and combinations thereof.

The solid material is preferably washed with water; sometimes some alkaline material or a surface-active material is added to the washing water. Also an organic solvents e.g. acetone can be used during washing. Drying preferably takes place with hot air. Spray-drying is preferred but freeze-drying is also quite possible.

The metal/metal compound aggregates of the catalyst consist mainly, i.e. for more than 80 or 90%, of cobalt and/or copper and their oxides, but some promotor material can also be present. These aggregates preferably contain metal crystallites having an average diameter between 10 and 25, more particularly between 14 and 20 nanometers.

The cobalt and/or copper catalysts so obtained are useful for the hydrogenation of unsaturated compounds and highly active and selective in the hydrogenation of higher nitriles to amines and semi-drying oils respectively.

The invention is illustrated by the following examples.

Example 1

An aqueous suspension was prepared by suspending kieselguhr (containing 58% amorphous $SiO_2$) in a cobalt nitrate hexahydrate solution (0.6 mol/litre), and in such a manner that the $SiO_2$/cobalt molecular ratio was 0.4. Further, as aqueous soda solution containing 100 g calcined soda per litre was prepared. Equal solutions of both solutions were continuously pumped into a vigorously stirred (about 25 Watts/kg solution) reactor, in which cobalt hydroxide/carbonate was precipitated at a temperature of 19°C. In the reactor in which the precipitation took place the suspension had a residence time of 30 seconds, whereafter the suspension was immediately pumped to an ageing reactor. In this reactor the precipitate was aged for 30 minutes (average residence time) at a temperature of 97°C. The aged precipitate was then filtered off in a rapid and continuous manner and the purple filter cake thus obtained was washed with water, spray-dried and activated with hydrogen at atmospheric pressure at a temperature of 450°C.

By X-ray fluorescence spectrometry it was determined that the cake contained 42.4% cobalt and 8.6% $SiO_2$ ($SiO_2/Co=0.43$). By electron microscopy it was established that the catalyst consisted of aggregates of an average of 14 micrometers. Micro X-ray analysis revealed that the cobalt crystallites had an average diameter of 16 nanometers. The cobalt/cobalt compound aggregates had a surface area of which about 85% was free of carrier particles, as is apparent from Figure I. The original shape of the infusoria skeletons were for the greater part free of precipitate and easily and freely perceptible. The active metal surface area was determined by hydrogen chemisorption and was found to be 9.2 $m^2$/g catalyst.

Example 2

By the same procedure as in Example 1 a copper catalyst on kieselguhr was prepared. The starting material was copper nitrate hexahydrate which, just like the anhydrous sodium carbonate, was used in the same molecular concentrations as in Example 1. However, ageing was carried out at 96°C, whereafter a blue cake was easily filtered off. After washing and spray-drying, the cake was activated in hydrogen at 250°C at 0.1 MPa. The cake contained 45.2% copper and 7.2% silica ($SiO_2/Cu=0.36$). The copper crystallite size was 16.5 nanometers and the aggregates 25 measured micrometers on an average. Here about 80% of the guhr particles appeared to have a surface area free of copper/copper compounds as is apparent from Figure II.

Carbon monoxide chemisorption was used to determine the active metal surface area and 2.2 $m^2$/g catalyst was found.

Examples 3 and 4

The activity of the cobalt-on-kieselguhr catalyst of Example 1 was tested in the hydrogenation reaction of $C_{18}$-nitrile to amine.

To this end an amount of activated Co-guhr catalyst, corresponding with amounts of Co-metal of 0.18 and 0.12% (based on the weight of the nitrile), were added to the nitrile.

The reaction was carried out in an autoclave in the presence of hydrogen and ammonia of which the partial pressures were 1.5 M Pa and 2.5 M Pa, respectively. When no more uptake of hydrogen was measured, the experiment was stopped. The conversion of nitrile into amine and the selectivity towards primary amine were determined. The conversion to amines was 70% and 100%, respectively, and the selectivity was 95% and 85%, respectively.

Example 5

The activity of the copper-on-kieselguhr catalyst prepared according to Example 2 was tested in the hydrogenation of soybean oil.

An amount of Cu-kieselguhr catalyst, corresponding with an amount of Cu metal of 0.3% (on the basis of the weight of the soybean oil), was added to the soybean oil. The activation of the Cu-kieselguhr catalyst by hydrogen took place in situ in the reaction vessel during the heating of the reaction mixture to the hydrogenation temperature.

The reaction was carried out in a hydrogenation vessel where, at atmospheric pressure, hydrogen (1 l/min) was stirred through the soybean oil with a stirrer (3000 rotations per minute). The reaction was carried out at a temperature of 185°C for 1 hour. The difference between the of refractive index of the starting material and of the reaction product was determined at a temperature of 65°C. This difference was taken as a yardstick for the activity of the catalyst. (Decrease of $N^{65}_D=1.4580$ to $N^{65}_D=1.4555$.)

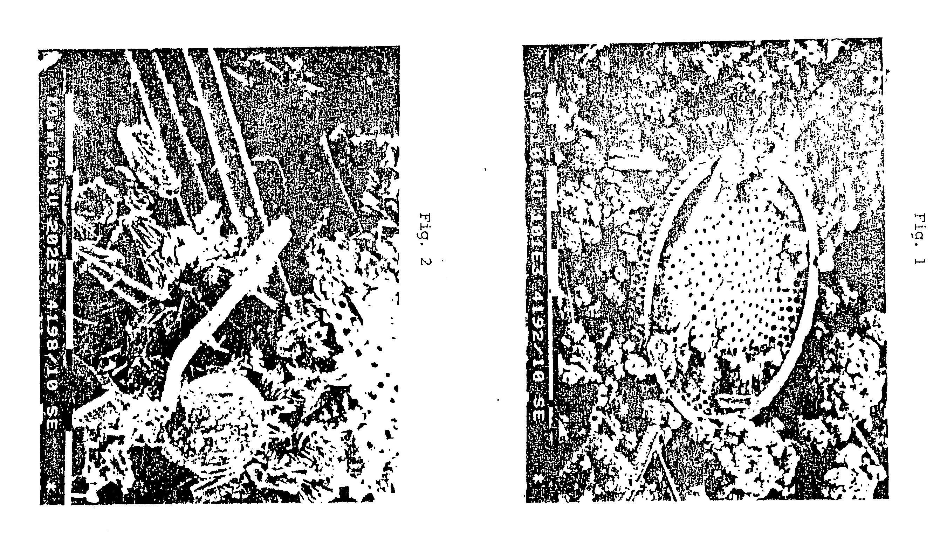

**Claims**

1. A hydrogenation catalyst which contains 10—90 parts by weight of a metal/metal compound and 90—10 parts by weight of silica and has a total active metal surface area of 1 to 20, preferably more than 2 $m^2$/g of metal and in which the metal has an uneven atomic-number between 26 and 30, characterized in that the catalyst contains metal/metal compound aggregates the average particle size of which is between 5 and 50 micrometers and surface area of the metal/metal compound aggregates are for at least 60% free of carrier particles.

2. A hydrogenation catalyst according to claim 1, characterized in that the surface area of the metal/metal compound aggregates is for at least 80% free of carrier particles.

3. A hydrogenation catalyst according to claim 1 or 2, characterized in that the surface area of the metal/metal compound aggregates are for at lesat 90% free of carrier particles.

4. A hydrogenation catalyst according to any one of the claims 1—3, characterized in that the average particle size of the metal/metal compound aggregates is between 10 and 30 micrometers.

5. A hydrogenation catalyst according to any one of the claims 1—4, characterized in that the average metal crystallite size is between 10 and 25 nanometers.

6. A hydrogenation catalyst according to any one of the claims 1—5, characterized in that the metal crystallite size is 14 to 20 nanometers.

7. A catalyst according to any one of the claims 1—6, characterized in that the metal has atomic number 27.

8. A process for the preparation of a catalyst containing a metal having an uneven atomic number between 26 and 30 as claimed in claim 1 which process comprises precipitation, ageing, separation and reduction and which catalyst contains as insoluble carrier and optionally a promotor, characterized in that the process is carried out in at least two separate steps, namely:

I. A rapid precipitation step in which, under vigorous stirring, metal hydroxide/carbonate is precipitated in a precipitation reactor in which the average residence time is 0.1 to 10 minutes, preferably between 0.2 and 4.5 minutes, more preferably less than 1.5 minutes, the normality of the solution in the reactor, which contains excess alkali, being between 0.05 and 0.5 N, preferably between 0.1 and 0.3 N, and the temperature of the liquid in the precipitation reactor being kept between 5 and 95, preferably between 15 and 55°C.

II. At least one separate, longer, ageing step with an average residence time in the ageing-reactor of 20 to 180 min., preferably between 30 and 120 min., and a temperature which remains between 60 and 100, preferably between 90 and 98°C, whereafter, by processes known in the art, the solid material is collected, dried and activated with hydrogen.

9. A process according to claim 8, characterized in that the metal salt solution contains 10—80 g metal per litre in the form of a soluble salt.

10. A process according to claim 8 or 9, characterized in that the carrier is added in an amount of 20—200 g per litre.

11. A process according to any one of the claims 8—10, characterized in that the solution of alkaline compound contains 30 to 300 g alkaline compound (calculated as anhydrous compound) per litre.

12. A process according to any one of the claims 8—11, characterized in that the alkaline solution contains sodium carbonate.

13. A process according to any one of the claims 8—12, characterized in that the metal compound is a salt of a mineral acid.

14. A process according to any one of the claims 8—13, characterized in that the carrier comprises silica.

15. A process according to claim 14, characterized in that the silica used is kieselguhr which consists for 50 to 90% by weight of amorphous $SiO_2$.

16. A process according to any one of the claims 8—14, characterized in that, during the precipitation, agitation takes place with a mechanical energy input of 5—2000 Watt per kilogram of solution.

17. A process according to any one of the claims 8—16, characterized in that the activation of the catalyst is carried out with hydrogen at a temperature between 150 and 500°C.

18. A process according to any one of the claims 8—17, characterized in that the precipitation is carried out continuously by dosing a carrier suspension into an aqueous metal salt solution and an alkaline solution together in a small, vigorously agitated mixing device and thereafter pumping the suspension into one or more post-reactors.

19. A process according to claim 18, characterized in that two or more post-reactors are used, the temperature in the second and possibly following post-reactor being 5—15°C lower than that in the first post-reactor.

20. A process for the hydrogenation of unsaturated compounds, characterized in that a catalyst according to any one of the claims 1—7 is used.

**Patentansprüche**

1. Hydrogenierungskatalysator, der 10 bis 90 Gew.-Teile einer Metall/Metallverbindung und 90 bis 10 Gew.-Teile Siliciumdioxid enthält und einen gesamten aktiven, metallischen Oberflächenbereich von 1 bis 20, vorzugeweise mehr als 2 $m^2$/g des Metalls aufweist und wobei das Metall eine ungerade Ordnungszahl zwischen 26 und 30 hat, dadurch gekennzeichnet, daß der Katalysator Metall/Metallverbindungsaggregate enthält, deren durchschnittliche Teilchengröße zwischen 5 und 50 Mikrometern liegt, und der Oberflächenbereich der Metall/Metallverbindungsaggregate zu mindestens 60% frei von Trägerteilchen ist.

2. Hydrogenierungskatalysator nach Anspruch 1, dadurch gekennzeichnet, daß der Oberflächenbereich der Metall/Metallverbindungsaggregate zu mindestens 80% frei von Trägerteilchen ist.

3. Hydrogenierungskatalysator nach Anspruch 1 oder 2, dadurch gekennzeichnet, daß der Oberflächenbereich der Metall/Metallverbindungsaggregate zu mindestens 90% frei von Trägerteilchen ist.

4. Hydrogenierungskatalysator nach irgendeinem der Ansprüche 1 bis 3, dadurch gekennzeichnet, daß die durchschnittliche Teilchengröße der Metall/Metallverbindungsaggregate zwischen 10 und 30 Mikrometern liegt.

5. Hydrogenierungskatalysator nach irgendeinem der Ansprüche 1 bis 4, dadurch gekennzeichnet, daß die durchschnittliche Metallkristallitgröße zwischen 10 und 25 Nanometern liegt.

6. Hydrogenierungskatalysator nach irgendeinem der Ansprüche 1 bis 5, dadurch gekennzeichnet, daß die Metallkristallitgröße zwischen 14 und 20 Nanometern liegt.

7. Hydrogenierungskatalysator nach irgendeinem der Ansprüche 1 bis 6, dadurch gekennzeichnet, daß das Metall eine Ordnungszahl von 27 aufweist.

8. Verfahren zur Herstellung eines Katalysators, der ein Metall einer ungeraden Ordnungszahl zwischen 26 und 30 enthält, wie im Anspruch 1 beansprucht, wobei dieses Verfahren das Ausfällen, das Altern, das Abtrennen und die Reduktion umfaßt und der Katalysator einen unlöslichen Träger und gegebenenfalls einen Promotor enthält, dadurch gekennzeichnet, daß das Verfahren in mindestens zwei gesonderten Stufen durchgeführt wird, nämlich:

I. in einer schnellen Ausfällungsstufe, in der ein Metallhydroxid/-carbonat in einem Ausfällungsreaktor unter kräftigem Rühren ausgefällt wird, in dem die durchschnittliche Verweilzeit 0,1 bis 10 Minuten beträgt, vorzugsweise 0,2 bis 4,5 Minuten, ganz besonders bevorzugt weniger als 1,5 Minuten, und wobei die Normalität der Lösung in dem Reaktor, die einen Überschuß an Alkali enthält, zwischen 0,05 und 0,5 N, vorzugsweise zwischen 0,1 und 0,3 N, liegt und die Temperatur der Flüssigkeit in dem Ausfällungsreaktor zwischen 5 und 95°C, vorzugsweise zwischen 15 und 55°C, gehalten wird;

II. in mindestens einer gesonderten längeren Alterungsstufe mit einer durchschnittlichen Verweilzeit in dem Alterungsreaktor von 20 bis 180 Minuten, vorzugsweise zwischen 30 und 120 Minuten, und einer Temperatur, die zwischen 60 und 100°C, vorzugsweise zwischen 90 und 98°C, bleibt, wonach das feste Material nach im Stand der Technik bekannten Verfahren gesammelt, getrocknet und mit Wasserstoff aktiviert wird.

9. Verfahren nach Anspruch 8, dadurch gekennzeichnet, daß die Metallsalzlösung 10 bis 80 g Metall pro Liter in Form eines löslichen Salzes enthält.

10. Verfahren nach Anspruch 8 oder 9, dadurch gekennzeichnet, daß der Träger in einer Menge von 20 bis 200 g pro Liter hinzugefügt wird.

11. Verfahren nach irgendeinem der Ansprüche 8 bis 10, dadurch gekennzeichnet, daß die Lösung der alkalischen Verbindung 30 bis 300 g alkalische Verbindung (berechnet als wasserfreie Verbindung) pro Liter enthält.

12. Verfahren nach irgendeinem der Ansprüche 8 bis 11, dadurch gekennzeichnet, daß die alkalische Lösung Natriumcarbonat enthält.

13. Verfahren nach irgendeinem der Ansprüche 8 bis 12, dadurch gekennzeichnet, daß die Metallverbindung ein Salz einer Mineralsäure ist.

14. Verfahren nach irgendeinem der Ansprüche 8 bis 13, dadurch gekennzeichnet, daß der Träger Siliciumdioxid umfaßt.

15. Verfahren nach Anspruch 14, dadurch gekennzeichnet, daß das verwendete Siliciumdioxid Kieselgur ist, die zu 50 bis 90 Gew.-% aus amorphem $SiO_2$ besteht.

16. Verfahren nach irgendeinem der Ansprüche 8 bis 14, dadurch gekennzeichnet, daß während des Ausfällens durch die Zufuhr mechanischer Energie von 5 bis 2000 Watt pro Kilogramm Lösung ein Rühren durchgeführt wird.

17. Verfahren nach irgendeinem der Ansprüche 8 bis 16, dadurch gekennzeichnet, daß die Aktivierung des Katalysators mit Wasserstoff bei einer Temperatur zwischen 150 und 500°C durchgeführt wird.

18. Verfahren nach irgendeinem der Ansprüche 8 bis 17, dadurch gekennzeichnet, daß das Ausfällen durch Eindosieren einer Trägersuspension in einer wäßrigen Metallsalzlösung zusammen mit einer alkalischen Lösung in eine kleine, kräftig gerührte Mischeinrichtung kontinuierlich durchgeführt und danach die Suspension in ein oder mehrere nachgeschaltete Reaktoren gepumpt wird.

19. Verfahren nach Anspruch 18, dadurch gekennzeichnet, daß zwei oder mehrere nachgeschaltete Reaktoren verwendet werden, wobei die Temperatur in dem zweiten und möglicherweise folgenden nachgeschalteten Reaktor 5 bis 15°C niedriger als in dem ersten nachgeschalteten Reaktor ist.

20. Verfahren zur Hydrogenierung ungesättigter Verbindungen, dadurch gekennzeichnet, daß ein Katalysator nach irgendeinem der Ansprüche 1 bis 7 verwendet wird.

**Revendications**

1. Catalyseur d'hydrogénation qui contient de 10 à 90 parties en poids d'un composé métal/métal et de 90 à 10 parties en poids de silice et qui a une aire specifique totale du métal actif de 1 à 20, de préférence de plus de 2 $m^2/g$ de métal, et dans lequel le métal possède un numéro atomique impair compris entre 26 et 30, caractérisé en ce que le catalyseur contient des agrégats du composé métal/métal dont la grosseur moyenne des particules est comprise entre 5 et 50 microns, et en ce que l'aire spécifique des agrégats du composé métal/métal est exempte pour au moins 60% de particules de support.

2. Catalyseur d'hydrogénation selon la revendication 1, caractérisé en ce que l'aire spécifique des agrégats du composé métal/métal est exempte pour au moins 80% de particules de support.

3. Catalyseur d'hydrogénation selon la revendication 1 ou 2, caractérisé en ce que l'aire spécifique des agrégats du composé métal/métal est exempte pour au moins 90% de particules de support.

4. Catalyseur d'hydrogénation selon l'une quelconque des revendications 1 à 3, caractérisé en ce que la grosseur moyenne des particules des agrégats du composé métal/métal est comprise entre 10 et 30 microns.

5. Catalyseur d'hydrogénation selon l'une quelconque des revendications 1 à 4, caractérisé en ce que la grosseur moyenne d'une cristallite du métal est comprise entre 10 et 25 nanomètres.

6. Catalyseur d'hydrogénation selon l'une quelconque des revendications 1 à 5, caractérisé en ce que la grosseur d'une cristallite du métal va de 14 à 20 nanomètres.

7. Catalyseur selon l'une quelconque des revendications 1 à 6, caractérisé en ce que le métal a le numéro atomique 27.

8. Procédé pour le préparation d'un catalyseur contenant un métal ayant un numéro atomique impair compris entre 26 et 30, selon la revendication 1, ce procédé comprenant la précipitation, le vieillissement, la séparation et la réduction et ce catalyseur contenant un support insoluble et éventuellement un promoteur, caractérisé en que l'on effectue le procédé en au moins deux étapes distinctes, à savoir:

I. Une étape de précipitation rapide dans laquelle, dans des conditions d'agitation énergique, l'hydroxyde de métal/carbonate précipite dans un réacteur de précipitation dans lequel le temps moyen de séjour est compris entre 0,1 et 10 minutes, de préférence entre 0,2 et 4,5 minutes, et est encore mieux inférieur à 1,5 minutes, la normalité de la solution dans le réacteur qui contient de l'alcali en excès étant comprise entre 0,05 et 0,5 N, de préférence entre 0,1 et 0,3 N, et la température du liquide dans le réacteur de préipitation étant maintenue entre 5 et 95, de préférence entre 15 et 55°C.

II. Au moins une étape de vieillissement, distincte, plus longue, avec un temps moyen de séjour dans le réacteur de vieillissement compris entre 20 et 180 minutes, de préférence entre 30 et 120 minutes, et à une température qui reste comprise entre 60 et 100, de préférence entre 90 et 98°C, après laquelle, par des procédés connus dans la technique, on recueille le matériau solide, on le sèche et on l'active avec de l'hydrogène.

9. Procédé selon la revendication 8, caractérisé en ce que la solution du sel métallique contient de 10 à 80 g de métal par litre, sous la forme d'un sel soluble.

10. Procédé selon la revendication 8 ou 9, caractérisé en ce l'on ajoute le support en une quantité de 20 à 200 g par litre.

11. Procédé selon l'une quelconque des revendications 8 à 9, caractérisé en ce que la solution du composé alcalin contient de 30 à 300 g de composé alcalin (calculé pour le composé anhydre) par litre.

12. Procédé selon l'une quelconque des revendications 8 à 11, caractérisé en ce que la solution alcaline contient du carbonate de sodium.

13. Procédé selon l'une quelconque des revendications 8 à 12, caractérisé en ce que le composé métallique est un sel d'un acide minéral.

14. Procédé selon l'une quelconque des revendications 8 à 13, caractérisé en ce que le support comprend de la silice.

15. Procédé selon la revendication 14, caractérisé en ce que la silice utilisée est du kieselguhr qui comprend de 50 à 90% en poids de $SiO_2$ amorphe.

16. Procédé selon l'une quelconque des revendications 8 à 14, caractérisé en ce que, au cours de la précipitation, on produit une agitation avec un apport d'énergie mécanique de 5 à 2000 watts par kilogramme de solution.

17. Procédé selon l'une quelconque des revendications 8 à 16, caractérisé en ce que l'on effectue l'activation du catalyseur avec de l'hydrogène à une température comprise entre 150 et 500°C.

18. Procédé selon l'une quelconque des revendications 8 à 17, caractérisé en ce que l'on effectue la précipitation en continu en dosant une suspension du support dans une solution aqueuse du sel métallique et une solution alcaline, simultanément dans un petit appareil de mélange soumis à une agitation énergique, et ensuite en pompant la suspension dans un ou plusieurs post-réacteurs.

19. Procédé selon la revendication 18, caractérisé en ce que l'on utilise deux post-réacteurs ou plus, la température dans le second post-réacteur et éventuellement les suivants étant de 5 à 15°C inférieure à celle régnant dans le premier post-réacteur.

20. Procédé pour l'hydrogénation de composés insaturés, caractérisé en ce que l'on utilise un catalyseur selon l'une quelconque des revendications 1 à 7.

0 168 096

Fig. 1

Fig. 2

1